Europäisches Patentamt

European Patent Office

Office européen des brevets

(11) Publication number: **0 475 205 A1**

# EUROPEAN PATENT APPLICATION

(21) Application number: **91114534.0**

(22) Date of filing: **28.08.91**

(51) Int. Cl.⁵: **A61K 7/13**

(30) Priority: **11.09.90 US 580694**
     **29.05.91 US 706671**

(43) Date of publication of application:
     **18.03.92 Bulletin 92/12**

(84) Designated Contracting States:
     **AT BE CH DE DK ES FR GB GR IT LI LU NL SE**

(71) Applicant: **BRISTOL-MYERS SOUIBB**
     **COMPANY**
     **345 Park Avenue**
     **New York, N.Y. 10154-0037(US)**

(72) Inventor: **Brown, Keith C.**
     **59 Douglas Road**
     **New Canaan, Connecticut 06840(US)**

(74) Representative: **Dipl.-Ing. H. Hauck, Dipl.-Ing.**
     **E. Graalfs, Dipl.-Ing. W. Wehnert, Dr.-Ing. W.**
     **Döring**
     **Mozartstrasse 23**
     **W-8000 München 2(DE)**

(54) Method for reducing scalp staining.

(57) The present invention provides an improvement in a process for dyeing hair on a live head wherein the hair is treated with metal ion in a pretreatment step to accelerate subsequent dyeing of the hair and the treated hair is then subjected to an oxidative or autooxidative dyeing step. The invention serves to reduce staining of the scalp occasioned by the dyeing step. The improvement comprises:(i) between the pretreatment step and the dyeing step the scalp of said live head is subjected to vigorous massage, rubbing, combing or brushing, for a time sufficient to reduce staining of the scalp occasioned by the dyeing step; or (ii) sufficient time is permitted to elapse between the pretreatment step and the dyeing step to reduce staining of the scalp occasioned by the dyeing step; or (iii) between the pretreatment step and the dyeing step the scalp of said live head is subjected to vigorous massage, rubbing, combing or brushing, and time is permitted to elapse between the pretreatment step and the dyeing step, the duration of the massage rubbing, combing or brushing coupled with the duration of the elapsed time between the pretreatment and dyeing steps being sufficient to reduce staining of the scalp occasioned by the dyeing step.

EP 0 475 205 A1

# BACKGROUND OF INVENTION

## RELATED APPLICATIONS

This application is a continuation-in-part of copending application Serial Number 580,694, filed September 11, 1990.

## FIELD OF INVENTION

The present invention relates to a method for reducing scalp staining when metal ion pretreatments are used to activate dyeing processes on live heads.

## DESCRIPTION OF THE PRIOR ART

The use of metal ion treatments to activate a variety of hair coloring processes is known in the art. For example Ger. 2,028,818 discloses accelerated oxidative dyeing by use of a pretreatment containing one of a variety of metal ions. Autooxidative dyeing systems which usually contain dye ingredients, such as pyrogallol, 1,2,-4-trihydroxybenzene or other readily oxidized phenolics, are also claimed to be activated by metal ions (see for example U.S. patent 629231).

Natural dyes and natural dye precursors that are known in the art as being capable of being activated by metal ions, include: indolic compounds (U.S. patents 2,934,296, and 3,194,734 and GB 797,174), logwood extracts (U.S. patent 4,801,302), henna and other quinone dyes (Czech 142,851).

Commercial exploitation of such dyeing processes is severely restricted by the fact that they have a number of significant drawbacks. The foremost drawback is the unacceptable amount of scalp staining produced when these dyeing systems are used on live heads. The affinity of metal ions for hair keratin and skin is quite similar. Therefore, a significant fraction of the metal ion applied is adsorbed by the scalp skin. Metal ion adsorbed by skin and metal adsorbed by hair have equivalent reactivity in the dyeing processes. Thus, a significant amount of color is produced on the scalp during hair dyeing. This is undesirable. With permanent dyes, i.e. dyes that are not readily removed by shampooing, the scalp coloration that is produced can last for a considerable time. Thus prior art processes are unsatisfactory.

The present invention provides an improvement in a process for dyeing hair on a live head wherein the hair is treated with metal ion in a pretreatment step to accelerate subsequent dyeing of the hair and the treated hair is then subjected to an oxidative or autooxidative dyeing step. The invention serves to reduce staining of the scalp occasioned by the dyeing step. The improvement comprises:

(a) between the pretreatment step and the dyeing step the scalp of said live head is subjected to vigorous massage, rubbing, combing or brushing, for a time sufficient to reduce staining of the scalp occasioned by the dyeing step; or

(b) sufficient time is permitted to elapse between the pretreatment step and the dyeing step to reduce staining of the scalp occasioned by the dyeing step; or

(c) between the pretreatment step and the dyeing step the scalp of said live head is subjected to vigorous massage, rubbing, combing or brushing, and time is permitted to elapse between the pretreatment step and the dyeing step, the duration of the massage rubbing, combing or brushing coupled with the duration of the elapsed time between the pretreatment and dyeing steps being sufficient to reduce staining of the scalp occasioned by the dyeing step.

In a preferred embodiment substantially immediately following the pretreatment step, the scalp of said live head is subjected to an interim step of vigorous massage, rubbing, combing or brushing, and the dyeing step is carried out from substantially immediately to less than 24 hours after the interim step. Preferably, the interim step is carried out substantially immediately following the pretreatment step. Another preferred process involves carrying out the dyeing step immediately following the interim step. Yet another preferred process involves carrying out the interim step substantially immediately following the pretreatment step and carrying out the dyeing step substantially immediately following the interim step.

## SUMMARY OF THE INVENTION

The present inventor found that the heretofore described undesirable scalp staining can surprisingly and unexpectedly be almost entirely eliminated by allowing a time period to elapse between application of the metal ion and application of the dye. The duration of the time period is dependent on a number of factors. Although the instant inventor does not wish to be restricted by the following hypothesis, it is believed that during such elapsed time period metal ion adsorbed onto the surface of the skin is desquamated along with the skin. Sufficient time must elapse for the natural process of desquamation to remove most of the active metal ions from the scalp skin. Generally, a period of at least 24 hours, more preferably a period of a least 48 hours of elapsed time, is adequate to reduce scalp staining to an acceptable level. As a practical matter, a period of 24 hours to less than 48 hours elapsed time will suffice but a period of 48 hours to 72 hours is optimal. During this elapsed time, the metal ion adsorbed onto the hair has not lost any of its activity with respect to activation of color formation.

Surprisingly and unexpectedly the present inventor has found that the aforementioned time delay is significantly reduced by processes which accelerate scalp desquamation. A vigorous shampooing after application of the metal ion-containing formulation allows the user to dye his or her hair using substantially less elapsed time between the pretreatment step and the dyeing step while obtaining a considerable reduction in concurrent scalp staining. For certain oxidative dyes a vigorous shampooing, after application of the metal ion-containing formulation allows the user to dye his or her hair substantially immediately afterward with a considerable reduction in concurrent scalp staining. Other processes which, like vigorous shampooing, substantially decrease the duration of the time period required between the application of the metal ion and the application of the dye include vigorous massaging, vigorous rubbing, vigorous combing and vigorous brushing of the scalp. Various combinations of these processes can be employed. Advantageously, none of these processes change the degree of dye take on the hair.

By vigorous, the present inventor means with sufficient intensity to cause at least partial exfoliation of the treated area. This requires much greater force than is utilized in normal shampooing, massaging, combing, brushing, etc.

The method of the present invention offers many advantages. Its primary advantage is that it allows a hair colorist to exploit the advantages of metal ion pretreatment for hair dyeing systems, i.e. faster development of color and more intense color formation, while substantially avoiding the usual concurrent problem of scalp staining.

**EXAMPLES**

Example 1

(a) A live head of gray hair was rinsed with a solution of 1 g of ferrous sulfate heptahydrate in 100 ml of water previously adjusted to pH 9.5. with a citric acid - ammonia mixture.

After a water rinse, a commercially available oxidative dye composition (NICE 'N EASY [R] Shade 120 - Dark Brown) was prepared following the package instructions then rubbed into the hair and left on for five minutes. It was then shampooed off. The hair was dyed dark brown. The scalp also showed strong staining, especially at the hair and part lines. The staining was not removed by shampooing.

(b) The procedure of part (a) of this Example 1 was repeated on another live head of gray hair, but this time 24 hours were allowed to elapse between the metal ion treatment step and the dyeing step. This time scalp staining was vastly less. Moreover, visible staining was readily removed by a shampooing.

Example 2

(a) A live head of gray hair was shampooed twice with a solution of copper sulfate (1g) in a 10% solution of sodium lauryl sulfate in water (100 ml).

After thorough rinsing, a freshly prepared solution of 5,6-dihydroxyindole (1g) in water (100 ml) was spread through the hair and left on for ten minutes. After rinsing and shampooing, the hair was dyed a deep black However, the scalp hair and part lines were also badly stained black. The staining was not removed by shampooing or rubbing.

(b) The procedure of part (a) of this Example 2 was repeated on another live head of gray hair, however, 24 hours were allowed to elapse between the metal ion and dihydroxyindole treatment steps. This time the scalp was almost free of any discoloration.

(c) A third head of live gray hair was dyed as described in part (b) of this Example 2 however, eight hours thereafter (with the interval between the metal ion and dihydroxyindole treatment steps the hair was vigorously shampooed with a commercial shampoo (CLAIROL CONDITION [R] for Normal Hair). After dyeing, there was no visible scalp staining.

Example 3

(a) A live head of white hair was rinsed with a solution of manganese chloride (2g) in (100 ml). The solution had been adjusted to pH 10 with monoethanolamine

After rinsing, a commercial auto-oxidative dye (CLAIROL OPTION GRADUAL [R] - Dark Brown: active ingredient 1,2,4-benzenetriol) was rubbed into the hair and left on for ten minutes. After rinsing and shampooing, the hair was colored dark brown and the scalp was heavily stained. The scalp staining could not be removed by shampooing or rubbing.

(b) A second head of live white hair was treated as described in part (a) of this Example 3, except that the metal ion solution was immediately followed by a vigorous shampoo with CLAIROL's CONDITION [R] Shampoo for Normal Hair.

After rinsing out the shampoo, the head was immediately dyed. This was followed by a rinse. After rinsing, almost no scalp staining was evident.

Example 4

To demonstrate that one can obtain the advantages of the present invention by combining a period of vigorous combing, or brushing, etc. with a waiting period less than that required for substantially complete exfoliating of the scalp to occur; the two periods being sufficient to enable enough exfoliation to occur such that scalp staining is reduced, the following experiments were carried out:

(a) a live head of hair (50% gray) was shampooed twice with a solution of cooper sulfate (1g) in a 10% solution of sodium Lauryl sulfate in water (100 ml). After rinsing, the hair was towel dried and then the hair and scalp were subjected to vigorous brushing with a commercial stiff bristle hair brush for about 10 minutes. During this time, particular attention was given to brushing the scalp around the hair line and front of the head where skin staining can be especially noticeable. At the end of this period, the hair was essentially dry and the scalp was quite stimulated. The hair was then parted centrally, so as to divide same into essentially two equal parts.

The left-hand side was now dyed for 5 minutes with a commercially available oxidative dye composition (CLAIROL NICE 'N EASY Shade 120-Dark Brown) prepared following the package instructions. After rinsing out the dye, it could be seen that the left-hand side of the hair was dyed dark brown and the scalp showed slight staining.

The right-hand side of the head was left essentially untouched for about 8 hours. It was then dyed exactly as described above. When this dye was rinsed out, the hair was seen to be dyed dark brown and there was no visible scalp staining. The right-hand scalp was clearly less stained that the left-hand side.

(b) In a second experiment, a live head was shampooed twice with the copper sulfate solution (of part (a) of this Example 4,) and then the whole head was immediately dyed for 5 minutes with NICE'N EASY Shade 120. After rinsing, it was evident that the hair was dyed dark brown, but the scalp especially at the hair and part lines was also significantly colored. In any event, the staining was much more noticeable than in either of the above experiments.

It is believed that the process of the present invention works because the metal ion absorbed onto the skin is exfoliated (desquamated) with the skin whereas the metal ion on the hair remains intact. Therefore, color formation, which needs the presence of metal ion, only occurs on the hair. Although desquamation of the top layer of skin takes up to several days, it is known that the process can be accelerated by washing and rubbing.

One does not have to achieve complete scalp desquamation in order to secure the benefits of the instant invention. All that is required is that the desquamation be sufficient to produce a significant reduction in the amount of visible skin staining. This does not require loss of all the metal ion from the scalp, just a significant reduction. Moreover, the metal ion is present only in the outermost layer and that layer is the first to be removed.

**Claims**

1. In a process for dyeing hair on a live head wherein the hair is treated with metal ion in a pretreatment step to accelerate subsequent dyeing of the hair and the treated hair is then subjected to an oxidative or autooxidative dyeing step, wherein the improvement comprises,

(a) between the pretreatment step and the dyeing step the scalp of said live head is subjected to vigorous massage, rubbing, combing or brushing, for a time sufficient to reduce staining of the scalp occasioned by the dyeing step; or

(b) sufficient time is permitted to elapse between the pretreatment step and the dyeing step to reduce staining of the scalp occasioned by the dyeing step; or

(c) between the pretreatment step and the dyeing step the scalp of said live head is subjected to vigorous massage, rubbing, combing or brushing, and time is permitted to elapse between the pretreatment step and the dyeing step, the duration of the massage rubbing, combing or brushing coupled with the duration of the elapsed time between the pretreatment and dyeing steps being sufficient to reduce staining of the scalp occasioned by the dyeing step.

2. The process, as claimed in claim 1, wherein at least 24 hours is permitted to elapse between the pretreatment step and the dyeing step.

3. The process, as claimed in claim 1, wherein at least 48 hours is permitted to elapse between the pretreatment step and the dyeing step.

4. The process, as claimed in claim 1, wherein from 24 to 72 hours is permitted to elapse between the pretreatment step and the dyeing step.

5. The process, as claimed in claim 1, wherein at least 48 to 72 hours is permitted to elapse between the pretreatment step and the dyeing step.

6. In a process for dyeing hair on a live head wherein the hair is treated with metal ion in a pretreatment step to accelerate subsequent dyeing of the hair and the treated hair is then subjected to an oxidative or autooxidative dyeing step, wherein the improvement comprises, following the pretreatment step the scalp of said live head is subjected to an interim step of vigorous massage, rubbing, combing or brushing and the dyeing step is carried out following the interim step and less than 24 hours after the pretreatment step whereby staining of the scalp occasioned by the dyeing step is reduced.

7. The process, as claimed in Claim 6, wherein the interim step is carried out substantially immediately following the pretreatment step.

8. The process, as claimed in Claim 6, wherein the dyeing step is carried out substantially immediately following the interim step.

9. The process, as claimed in Claim 6, wherein the interim step is carried out substantially immediately following the pretreatment step and the dyeing step is carried out substantially immediately following the interim step.

European
Patent Office

EUROPEAN SEARCH
REPORT

Application Number

EP 91 11 4534

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 341 808   (BRISTOL-MYERS CO.)<br>* Tables I-III; claims 1-4,6-14 *<br>– – – | 1,6 | A 61 K 7/13 |
| A | GB-A-2 192 645   (L'OREAL)<br>* Examples; claims *<br>– – – | 1,6 | |
| A | GB-A-2 132 642   (BRISTOL-MYERS CO.)<br>* Examples; claims *<br>– – – | 1,6 | |
| A | DE-A-2 028 818   (THE GILLETTE CO.)<br>* Examples; claims *<br>– – – – – | 1,6 | |

TECHNICAL FIELDS
SEARCHED (Int. Cl.5)

A 61 K

The present search report has been drawn up for all claims

| Place of search | Date of completion of search | Examiner |
|---|---|---|
| The Hague | 04 November 91 | COUCKUYT P.J.R. |